(19) Europäisches Patentamt
European Patent Office
Office européen des brevets

(11) EP 1 683 571 A1

(12) EUROPEAN PATENT APPLICATION

(43) Date of publication:
26.07.2006 Bulletin 2006/30

(51) Int Cl.:
*B01J 19/00* (2006.01) *G01N 33/553* (2006.01)
*C23C 18/16* (2006.01) *H05K 3/38* (2006.01)

(21) Application number: 06000607.9

(22) Date of filing: 12.01.2006

(84) Designated Contracting States:
AT BE BG CH CY CZ DE DK EE ES FI FR GB GR HU IE IS IT LI LT LU LV MC NL PL PT RO SE SI SK TR
Designated Extension States:
AL BA HR MK YU

(30) Priority: 20.01.2005 KR 2005005532

(71) Applicant: Samsung Electronics Co., Ltd.
Suwon-si, Gyeonggi-Do (KR)

(72) Inventors:
- Lee, In-ho
dong,
Giheung-gu,
Yongin-si,
Gyeonggi-do (KR)
- Min, Jung-hong
Jukjeon-dong,
Yongin-si,
Gyeonggi-do (KR)
- Kim, Su-hyeon
Seoul (KR)
- Kim, Kui-hyun
Daejeon-si (KR)
- Yang, Seung-yeon,
904-1704 Cheongsol Maeul Jugong
Bundang-gu,
Seongnam-si,
Gyeonggi-do (KR)

(74) Representative: Grünecker, Kinkeldey, Stockmair & Schwanhäusser
Anwaltssozietät
Maximilianstrasse 58
80538 München (DE)

(54) **Patterning method, substrate for biomolecule immobilization using the patterning method, and biochip employing the substrate**

(57) Provided is a linker functional group patterning method for biomolecule immobilization. The patterning method includes preparing a coating composition including a hydrophobic group-containing silane compound and a hydrophilic group-containing silane compound; forming a surface tension control layer by coating the coating composition on a substrate for biomoleucle immobilization; and forming a linker functional group pattern on the surface tension control layer using a coating composition including a linker functional group-containing compound followed by thermal treatment. The linker functional group pattern is formed in a uniform size and distribution and contains high-density linker functional groups.

FIG. 4

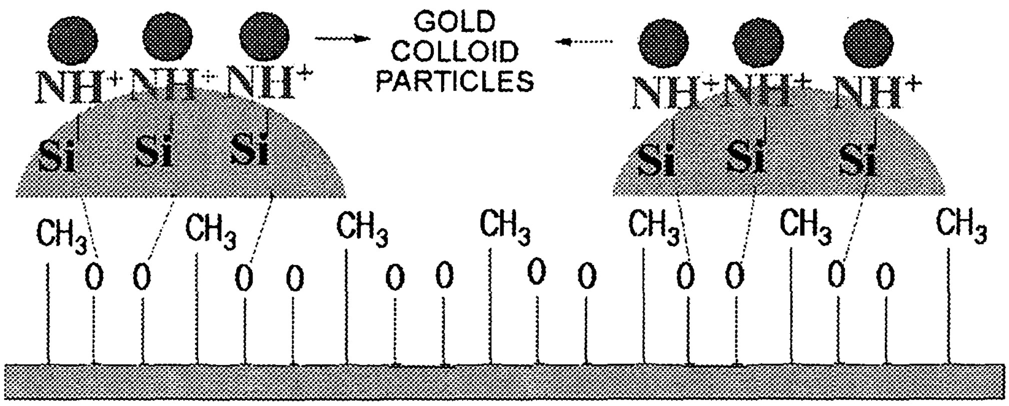


EP 1 683 571 A1

**Description**

1. Field of the Invention

The present invention relates to a metal thin film patterning method, a substrate for biomolecule immobilization using the patterning method, and a biochip employing the substrate. More particularly, the present invention relates to a patterning method capable of forming a metal thin film with a uniform size and distribution on a substrate, a substrate for biomolecule immobilization using the patterning method, and a biochip employing the substrate.

2. Description of the Related Art

Recently, efforts to find the activities of biomolecules such as nucleic acids, proteins, enzymes, antigens, and antibodies through the fusion of biotechnology and semiconductor fabrication technology have been made throughout the world. Overall biochemical analysis of biochips where desired biomolecules are immobilized on specific zones of a silicon microchip using a semiconductor processing technique can easily provide important inflormation.

Biochips are semiconductor chip-type devices made from combination of organic biomolecules derived from biological organism, such as enzymes, proteins, antibodies, DNAs, microorganism, animal/plant cells and organs, nerve cells and organs, and inorganic substances such as semiconductors. Biochips can be largely classified into "DNA chips" immobilized with DNA probes, "protein chips" immobilized with proteins such as enzymes, antibodies, and antigens, and automated "lab-on-a-chip" integrating sample pretreatment, biochemical reaction, detection, and data analysis onto one microchip.

To develop such biochips, it is important to develop a biomolecule immobilization technique capable of efficiently forming an interface between biomolecules and a substrate and maximally using the intrinsic functions of the biomolecules. Immobilization of biomolecules occurs on surfaces of slide glasses, silicon wafers, microwell plates, tubes, spherical particles, and porous films. Various attempts to improve a contact between a substrate surface and ends of biomolecules have been made. For example, DNA immobilization may be performed by activating 5'-phosphate groups of DNAs with carbodiimide and reacting the activated DNAs with functional groups of a substrate surface.

U.S. Patent No. 5,858,653 discloses a composition including a polymer having a thermochemically reactive group or a photoreactive group to react a surface of a substrate with one or more ion groups reactive with biomolecules, e.g., quaternary ammonium salt, hydrogenated tertiary amine, or phosphonium. U.S. Patent No. 5,981,734 discloses that DNA immobilization on an amino or aldehyde group-containing polyacrylamide gel facilitates biochemical assay due to stable hybridization. U.S. Patent No. 5,869,272 discloses a method of detecting bacterial antigen based on change in optical characteristics (color) of an optically active surface between an attachment layer and a protein layer. Here, the attachment layer is formed on a silicon wafer by spin-coating aminosilane and is made of a dendrimer, a star polymer, a self-assembly polymer, polysiloxane, latex, or the like. U.S. Patent No. 5,919,523 discloses a method of forming a linker layer by treating an aminosilane-coated substrate with glycine, serine, or an amine-, imine-, or amide-based organic polymer.

These patent documents relate to formation of an aminosilane self-assembly monolayer and has difficulty in uniformly maintaining the surface density of linker functional groups. Furthermore, it is difficult to control the patterning of linker functional groups, thereby leading to the immobilization of biomolecules on unwanted regions.

U.S. Patent No. 5,985,551 discloses a method of forming predetermined-shaped DNA spots by coating a solid substrate with aminosilane and forming a hydrophilic surface on regions of the solid substrate intended for DNA immobilization and a hydrophobic fluorosiloxane surface on the other regions of the solid substrate using photolithography. According to this method, coating regions made of a linker functional group-containing compound are separated from each other by the hydrophobic surface, which makes it easy to control a surface density of the linker functional groups. However, there arise problems in that the photolithography method is complicated and takes a long time, initial costs are excessively incurred, and mass production is difficult.

Biomolecular assay can also be performed on nanopores. In this case, a surface treatment is required to prevent attachment of biomolecules onto surfaces around the nanopores. In particular, immobilization of bioprobes on selected regions around the nanopores is required. For this, a gold pattern may be formed around the nanopores to immobilize biomolecules on the selected regions. In this case, when the gold pattern is formed by photolithography, it is necessary to use room temperature and atmospheric pressure conditions because there is a likelihood of damage to the nanopores occurring under photolithographic deposition environment.

A gold pattern can also be formed by microstamping which is an electroless plating method. In this case, however, it is difficult to control a surface pattern, stamp fabrication requires a separate photolithography process, and a stamp mediates a low-level adhesion between gold and a substrate.

## SUMMARY OF THE INVENTION

The present invention provides a patterning method for uniformly forming a high-density metal pattern capable of binding with a biomolecule.

The present invention also provides a substrate for biomolecule immobilization using the patterning method.

The present invention also provides a biochip employing the substrate.

According to an aspect of the present invention, there is provided a patterning method including:

forming on a substrate a pattern control layer including a hydrophobic group-containing silane compound and a hydrophilic group-containing silane compound;
forming a selectively patterned ion interaction layer on the pattern control layer;
forming a seed colloid particle layer on the patterned ion interaction layer; and
growing a metal thin film from the seed colloid particle layer.

The hydrophobic group-containing silane compound may be a compound represented by formula 1 below:

$$X\text{-}Si(R_1)_3, \qquad (1)$$

wherein X is a hydrophobic group, and $R_1$ is hydrogen, a substituted or unsubstituted alkoxy of 1-20 carbon atoms, or halogen.

The hydrophobic group of formula 1 may be a substituted or unsubstituted alkyl group of 1-20 carbon atoms or a substituted or unsubstituted aryl group of 6-30 carbon atoms.

The hydrophobic group-containing silane compound may be octadecyltrichlorosilane or octadecyltriethoxysilane as an alkyl group-containing silane compound; phenyloxyundecyltrimethoxysilane as an aryl group-containing silane compound; or (heptadecafluoro-1,1,2,2-tetrahydrodecyl)triethoxysilane as a halogen-substituted alkyl group-containing silane compound.

The hydrophilic group-containing silane compound may be a compound represented by formula 2 below:

$$Y\text{-}Si(R_2)_3, \qquad (2)$$

wherein Y is a hydrophilic group, and $R_2$ is hydrogen, a substituted or unsubstituted alkoxy of 1-20 carbon atoms, or halogen.

The hydrophilic group of formula 2 may be a substituted or unsubstituted hydroxyalkyl group of 1-20 carbon atoms, a substituted or unsubstituted carboxyalkyl group of 2-20 carbon atoms, a substituted or unsubstituted hydroxyalkylamino group of 1-20 carbon atoms, a substituted or unsubstituted hydroxyalkylaminoalkyl group of 2-20 carbon atoms, a substituted or unsubstituted di(hydroxyalkyl)aminoalkyl group of 3-20 carbon atoms, or a substituted or unsubstituted di(hydroxy)alkylaminoalkyl group of 2-20 carbon atoms.

In the pattern control layer, the weight ratio of the hydrophobic group-containing silane compound to the hydrophilic group-containing silane compound may range from 0.9:0.1 to 0.3:0.7.

The ion interaction layer may include a compound represented by formula 3 below:

$$(R_3)_3\text{-}Si\text{-}Z, \qquad (3)$$

wherein $R_3$ is hydrogen, a substituted or unsubstituted alkoxy of 1-20 carbon atoms, or halogen, and Z is a positively charged functional group.

The ion interaction layer may be formed on the pattern control layer by piezoelectric printing, micropipetting, inkjet printing, spotting, or stamping.

Seed colloid particles of the seed colloid particle layer may be gold colloid particles having an average particle size of 5 to 10 nm.

The metal thin film may be a gold thin film.

The substrate may be a glass, a silicon wafer, polycarbonate, polystyrene, or polyurethane.

Forming the pattern control layer may be performed using a coating composition including a surface tension controlling compound composed of the hydrophobic group-containing silane compound and the hydrophilic group-containing silane compound and a coating solvent.

The coating composition may be coated on the substrate by a wet coating method selected from the group consisting of dipping, spraying, spin-coating, and printing.

According to another aspect of the present invention, there is provided a substrate for biomolecule immobilization including:

a pattern control layer, formed on a base substrate, controlling a surface tension;
a patterned ion interaction layer formed on the pattern control layer; and
a metal thin film selectively formed on the patterned ion interaction layer.

The substrate for biomolecule immobilization may have 1 to 10,000 biomolecule binding sites per $cm^2$, and each biomolecule binding site may have a diameter of 50 to 5,000 $\mu$m.
According to still another aspect of the present invention, there is provided a biochip patterned by reacting and binding a biomolecule or a functional group-activated biomolecule with the metal thin film of the substrate for biomolecule immobilization.
The biomolecule may be at least one selected from the group consisting of enzymes, proteins, antibodies, microorganism, animal cells and organs, plant cells and organs, nerve cells, DNAs, and RNAs, derived from living species or equivalents thereof, or synthesized *ex vivo*.
The patterning of the biochip may be performed by photolithography, piezoelectric printing, micropipetting, or spotting.

## BRIEF DESCRIPTION OF THE DRAWINGS

The above and other features and advantages of the present invention will become more apparent by describing in detail exemplary embodiments thereof with reference to the attached drawings in which:

FIG. 1 is a schematic diagram illustrating a pattern control layer of Example 1;
FIG. 2 is a schematic diagram illustrating an ion interaction layer formed on the pattern control layer of Example 1;
FIG. 3 illustrates a gold colloid particle used in Example 1, a surface of which is attached with ionic functional groups;
FIG. 4 is a schematic diagram illustrating gold colloid particles linked to the ion interaction layer of Example 1;
FIG. 5 is a graph illustrating self-emission intensities of ion interaction layers of Examples 1-5 and Comparative Examples 1-2;
FIG. 6 is photographic images showing substrate surfaces after electroless plating according to Examples 1-5 and Comparative Examples 1-2;
FIG. 7 is photographic scans showing the substrate surface of Example 1 after forming an ion interaction layer, after coating gold colloid particles, and after electroless plating;
FIG. 8 is an optical microscopic image showing a gold thin film pattern formed in Example 1;
FIG. 9 is photographic images showing surface morphologies of gold thin film patterns of Examples 1 and 6-8, with respect to the reaction time between an ion interaction layer and gold colloid particles; and
FIG. 10 are photographic images showing particle growth with respect to the size of gold colloid particles used in Examples 1 and 9.

## DETAILED DESCRIPTION OF THE INVENTION

The present invention will now be described more fully with reference to the accompanying drawings, in which exemplary embodiments of the invention are shown.
The present invention provides a patterning method including: forming, on a substrate, a pattern control layer including a hydrophobic group-containing silane compound and a hydrophilic group-containing silane compound; forming a selectively patterned ion interaction layer on the pattern control layer; forming a seed colloid particle layer on the patterned ion interaction layer; and growing a metal thin film from the seed colloid particle layer.
The patterning method can be used in fabrication of a substrate for a biochip for optical and electrical measurement, and in particular, can be usefully used in the fields having difficulty in photolithographic patterning, e.g., nanopores or three-dimensional structures.
The patterning method includes forming on the substrate the pattern control layer including the hydrophobic group-containing silane compound and the hydrophilic group-containing silane compound. The pattern control layer can control a surface tension of the substrate and is formed on the entire surface of the substrate to control the shape, size, etc. of a pattern to a desired level. For the pattern control layer, a mixture obtained by mixing the hydrophobic group-containing silane compound and the hydrophilic group-containing silane compound in a predetermined ratio is applied to the substrate. The hydrophobic group-containing silane compound serves to impart hydrophobicity to a surface of the substrate and the hydrophilic group-containing silane compound forms a covalent bond with the patterned ion interaction layer in a subsequent process.
At this time, it is important to adjust a mixture ratio of the hydrophobic group-containing silane compound and the hydrophilic group-containing silane compound in the pattern control layer to form a covalent linkage between the pattern control layer and the ion interaction layer while maintaining a pattern shape of the ion interaction layer. Thus, it

is preferable to mix the hydrophobic group-containing silane compound and the hydrophilic group-containing silane compound in a weight ratio of 0.9:1 to 0.3:0.7, more preferably 0.8:0.2 to 0.5:0.5, and most preferably 0.7:0.3. If the weight ratio of the hydrophobic group-containing silane compound and the hydrophilic group-containing silane compound is outside the above range, undesirable results may be caused. That is, if the ratio of the hydrophobic group-containing silane compound relative to the hydrophilic group-containing silane compound is excessively high, attachment of the ion interaction layer to the pattern control layer may not occur, resulting in no formation of a metal pattern. On the other hand, if the ratio of the hydrophilic group-containing silane compound relative to the hydrophobic group-containing silane compound is excessively high, the ion interaction layer may be formed on the entire surface of the substrate, thereby leading to formation of metal on the ensure surface of the substrate, resulting in no formation of a desired pattern.

The hydrophobic group-containing silane compound and the hydrophilic group-containing silane compound are added to a coating solvent in the above ratio to prepare a coating composition. The coating composition may be applied onto the substrate by a wet-coating method selected from the group consisting of dipping, spraying, spin-coating, and printing. Since the pattern control layer can be simply formed by a wet-coating method, the present invention can greatly reduce a process duration relative to a conventional photolithography process.

The coating solvent may be a mixed solvent of water and an organic solvent. The organic solvent may be a water-compatible organic solvent such as an alcohol solvent (e.g., methanol, ethanol, propanol, and butanol), a cellosolve solvent (e.g., methyl cellosolve), dimethylformamide, or an acetone. A mixture of two or more organic solvents may also be used.

The coating composition includes a compound for forming the pattern control layer, i.e., the hydrophobic group-containing silane compound and the hydrophilic group-containing silane compound in an amount of 0.1 to 90 wt%, preferably 1 to 50 wt%. If the total content of the hydrophobic group-containing silane compound and the hydrophilic group-containing silane compound is less than 0.1 wt%, it may be difficult to form an appropriate pattern control layer. On the other hand, if it exceeds 90 wt%, it may be difficult to assure coating property.

The hydrophobic group-containing silane compound may be a compound represented by formula 1 below:

$$X\text{-}Si(R_1)_3, \qquad (1)$$

wherein X is a hydrophobic group, and $R_1$ is hydrogen, a substituted or unsubstituted alkoxy of 1-20 carbon atoms, or halogen.

The hydrophobic group of formula 1 may be a substituted or unsubstituted alkyl group of 1 to 20 carbon atoms or a substituted or unsubstituted aryl group of 6 to 30 carbon atoms, but the present invention is not limited thereto. The substituted alkyl group of 1-20 carbon atoms may be a halogen-substituted alkyl group of 1 to 20 carbon atoms and the aryl group may be a phenyl group.

The hydrophobic group-containing silane compound may be octadecyltrichlorosilane or octadecyltriethoxysilane as an alkyl group-containing silane compound; phenyloxyundecyltrimethoxysilane as an aryl group-containing silane compound; or (heptadecafluoro-1,1,2,2-tetrahydrodecyl)triethoxysilane as a substituted alkyl group-containing silane compound.

The hydrophilic group-containing slane compound may be a compound represented by formula 2 below:

$$Y\text{-}Si(R_2)_3, \qquad (2)$$

wherein Y is a hydrophilic group, and $R_2$ is hydrogen, a substituted or unsubstituted alkoxy of 1-20 carbon atoms, or halogen.

The hydrophilic group of formula 2 may be a substituted or unsubstituted hydroxyalkyl group of 1-20 carbon atoms, a substituted or unsubstituted carboxyalkyl group of 2-20 carbon atoms, a substituted or unsubstituted hydroxyalkylamino group of 1-20 carbon atoms, a substituted or unsubstituted hydroxyalkylaminoalkyl group of 2-20 carbon atoms, a substituted or unsubstituted di(hydroxyalkyl)aminoalkyl group of 3-20 carbon atoms, or a substituted or unsubstituted di(hydroxy)alkylaminoalkyl group of 2-20 carbon atoms.

The hydrophilic group-containing silane compound may be bis(2-hydroxyethyl)-3-aminopropyltriethoxysilane, cyanoethyltrimethoxysilane, etc.

After forming the pattern control layer on the substrate, the selectively patterned ion interaction layer is formed on the pattern control layer. The ion interaction layer serves as a linker mediating the binding between the pattern control layer and seed colloid particles, and its binding aspect depends on the composition of the pattern control layer. That is, when the content of the hydrophobic group-containing silane compound increases, an adhesion between the pattern control layer and the ion interaction layer may decrease, which makes it difficult to form a desired pattern. If the content of the hydrophilic group-containing silane compound increases, the ion interaction layer may be formed on the entire surface of the substrate, which makes it difficult to form a desired pattern. The composition ratio of the hydrophobic group-containing silane compound to the hydrophilic group-containing silane compound is as described above.

The ion interaction layer may be patterned on the pattern control layer by piezoelectric printing, micropipetting, inkjet printing, or spotting, but the present invention is not limited thereto. According to the above-described method, the ion interaction layer can be selectively formed on specified regions, and thus, a desired pattern form, i.e., a desired pattern size and shape can be appropriately controlled. The ion interaction layer ionically interacts with seed colloid particles in a subsequence process to attach the seed colloid particles onto only the ion interaction layer. That is, when the ion interaction layer is patterned on the pattern control layer wholly covering the substrate, seed colloid particles ionically interact with only the surface of the ion interaction layer to attach the seed colloid particles onto only the surface of the ion interaction layer. The surface of the pattern control layer on which the ion interaction layer is absent cannot ionically interact with the seed colloid particles to prevent the attachment of the seed colloid particles to the substrate.

A material forming the ion interaction layer may be a compound represented by formula 3 below:

$$(R_3)_3\text{-Si-Z}, \qquad (3)$$

wherein $R_3$ is hydrogen, a substituted or unsubstituted alkoxy of 1-20 carbon atoms, or halogen, and Z is a positively charged functional group.

The compound of formula 3 has a positively charged functional group capable of binding with negatively charged seed colloid particles. The positively charged functional group may be $-NH^+$ and its counter ion may be a halogen atom. The compound of formula 3 having the positively charged functional group may form a polymer by binding of positively charged functional groups. The polymeric compound of formula 3 may be trimethoxysilylpropyl(polyethyleneimine), gamma-propyltriethoxysilane, etc.

The compound of formula 3 may be a polymer having a repeat unit of formula 3a below:

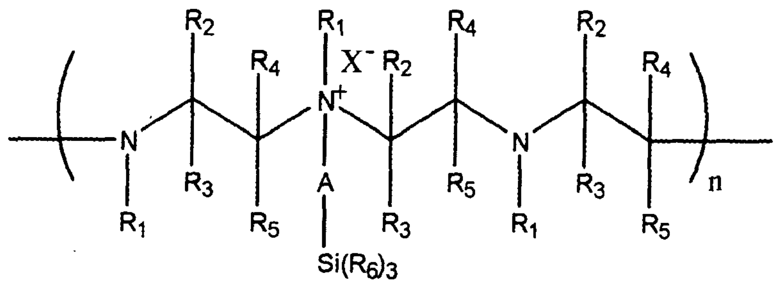

(3a)

wherein $R_1$ to $R_5$ are each independently a substituted or unsubstituted alkyl group of 1-20 carbon atoms or a substituted or unsubstituted aryl group of 6-30 carbon atoms, and $R_6$ is hydrogen, a substituted or unsubstituted alkoxy group of 1-20 carbon atoms, or halogen.

The ion interaction layer can be formed using the above compound in itself or a diluted solution of the above compound in a solvent. For this, there may be used a water-compatible organic solvent such as an alcohol solvent (e.g., methanol, ethanol, propanol, and butanol), a cellosolve solvent (e.g., methyl cellosolve), dimethylformamide, or an acetone. A mixture of two or more organic solvents may also be used.

After forming the ion interaction layer, the seed colloid particle layer is formed on the ion interaction layer. This is a pretreatment process for subsequent electroless plating to previously form seed colloid particles on the ion interaction layer. Thus, a desired metal thin film can be grown from the seed colloid particles in a subsequence process.

The seed particles forming the seed colloid particle layer may be metal particles having ionic functional groups on surfaces thereof, preferably, gold particles having -COO-(carboxy) groups on surfaces thereof. The seed particles are applied onto the substrate in the form of a colloid.

The seed colloid particles can be prepared by reduction of a metal salt in the presence of a reducing agent. When the substrate on which the ion interaction layer is formed is dipped in a colloid solution containing the seed colloid particles for a predetermined time, a positively charged functional group of the ion interaction layer ionically interacts with a negatively charged functional group of the seed colloid particles to thereby form the seed colloid particle layer on the ion interaction layer.

In particular, an effect of the particle size of the seed colloid particles on the growth rate of the seed colloid particles can be explained by the Gibbs-Thomson equation below:

$$\Delta E = \frac{2\sigma V_m}{\Gamma F}$$

where $\Delta$ E = free energy of particles, $\sigma$ = free surface energy, $V_m$ = volume per mole, $\Gamma$ = dimension of particles, and F = Faraday constant.

In the above equation, the free energy of particles is in inverse proportion to the size of the particles. As a particle size increases, a particle growth rate decreases. Thus, it is important to select the seed colloid particles with an appropriate particle size. In particular, as the particle size of the seed colloid particles increases, more pores can be found on the surface. This can be explained by the size and number of pores formed between particles being changed according to a particle size, thereby changing a particle growth rate.

In this regard, the particle size of the seed colloid particles and reaction duration act as important process factors. Preferably, the seed colloid particles may have an average particle size of 1 to 30 nm, and particularly preferably 5 to 8 nm. If the particle size of the seed colloid particles is outside the above range, it may be difficult to control a metal thin film to a nanoscale thickness.

The substrate may be dipped in the colloid solution for 0.01 to 12 hours, and particularly preferably 0.1 to 1 hour. When the dipping time increases within the above range, small particles are uniformly and densely arranged, thereby enhancing surface roughness of the substrate. If the dipping time is less than 0.01 hours, the seed colloid particles may not sufficiently interact with the ion interaction layer. On the other hand, if it exceeds 12 hours, cost effectiveness may be lowered.

After forming the seed colloid particle layer on the ion interaction layer, the metal thin film is grown from the seed colloid particle layer. This process is to attach metal onto surfaces of the seed colloid particles. The metal wholly surrounds the seed colloid particles in the form of a thin film. That is, the seed colloid particles are selectively attached to the patterned ion interaction layer, and thus have a predetermined patterned shape. Accordingly, the metal is formed in the form of a thin film on the seed colloid particles.

In the formation of the metal thin film, a plating method, a plating time, the concentration of a reducing agent used, etc. act as important process factors. Preferably, electroless plating may be used. The electroless plating may be performed by dipping the substrate on which the seed colloid particle layer is formed in a plating solution for a predetermined time. Preferably, the plating solution may be prepared by adding a desired metal salt and a reducing agent in a solvent. For example, to form a gold thin film, $HAuCl_4 \cdot 3H_2O$ may be used as the metal salt and $NH_2OH \cdot HCl$ may be used as a reducing agent.

The electroless plating may be performed for 3 to 20 minutes. If the plating time is less than 3 minutes, the thin film may not be sufficiently formed. On the other hand, if it exceeds 20 minutes, the metal salt may form particles in a solution, which renders further growth of a metal thin film difficult, and surface roughness may be lowered.

The substrate used in the patterning method according to the present invention may be a glass, a silicon wafer, polycarbonate, polystyrene, or polyurethane, but the present invention is not limited thereto. The substrate may be in the form of a microwell plate, a tube, a spherical particle, or a porous film.

The present invention also provides a substrate for biomolecule immobilization including a pattern control layer formed on a base substrate; an ion interaction layer patterned on the pattern control layer; and a metal thin film selectively formed on the ion interaction layer.

The metal thin film is formed on selected regions of the substrate at room temperature under atmospheric pressure to immobilize biomolecules on the selected regions. Therefore, the substrate for biomolecule immobilization enables efficient electrical and optical measurement due to more precision and less damage.

The pattern control layer, the ion interaction layer, and the metal thin film used as main constitutional elements of the substrate for biomolecule immobilization are as described above in the patterning method, and can be easily formed according to respective processes of the patterning method.

The substrate for biomolecule immobilization may have 1 to 10,000 biomolecule binding sites per $cm^2$, and each biomolecule binding site may have a diameter of 50 to 5,000 $\mu$m.

In particular, when a nanopore-containing substrate is used as the base substrate, the substrate for biomolecule immobilization may include an ion interaction layer formed on a pattern control layer and selectively patterned around nanopores; and a metal thin film selectively formed on the ion interaction layer patterned around the naopores.

The present invention also provides a biochip patterned by reacting and binding a biomolecule or a functional group-activated biomolecule with the metal thin film of the substrate for biomolecule immobilization.

The patterning of the biochip may be performed by a patterning method selected from the group consisting of piezoelectric printing, micropipetting, and spotting.

As used herein, the term "biomolecule" comprehends enzymes, proteins, antibodies, microorganism, animal

cells and organs, plant cells and organs, nerve cells, DNAs, RNAs, etc., derived from living species or equivalents thereof, or synthesized *ex vivo.* More preferably, the biomolecule may be DNA, RNA, or protein. Here, examples of the DNA include cDNA, genomic DNA, and oligonucleotide, examples of the RNA include genomic RNA, mRNA, and oligonucleotide, and examples of the protein include antibody, antigen, enzyme, and peptide.

Hereinafter, the present invention will be described more specifically with reference to the following examples. The following examples are for illustrative purposes and are not intended to limit the scope of the invention.

Example 1

(1-a) Formation of pattern control layer

0.7 g of octadecyltrichlorosilane (OTS) of formula 4 below and 0.3 g of bis(2-hydroxyethyl)-3-aminopropyltriethoxysilane of formula 5 below were mixed with 99g of ethanol used as a coating solvent to prepare a coating composition for forming a pattern control layer. At this time, the weight ratio of the compound of formula 4 to the compound of formula 5 was 7:3. A slide glass was coated with the coating composition by dipping.

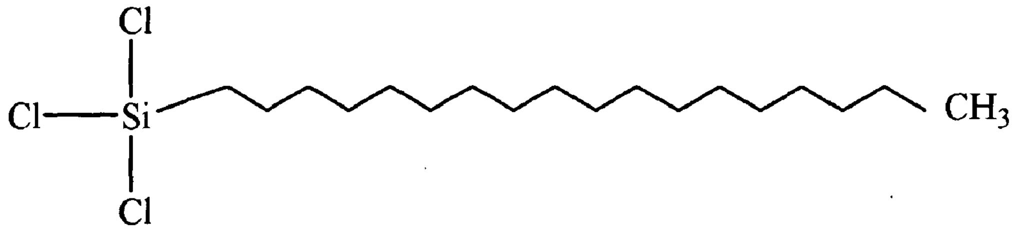

(4)

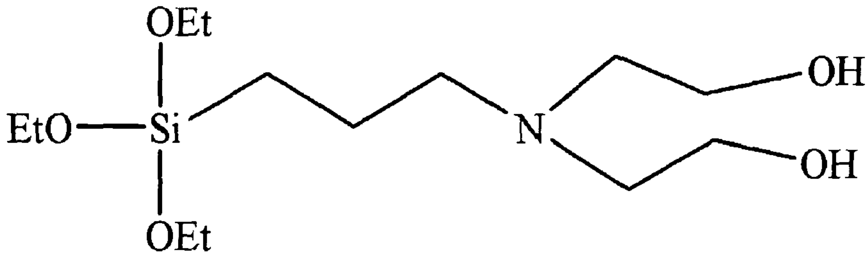

(5)

(1-b) Formation of ion interaction layer pattern

An ion interaction layer pattern containing a positively charged functional group capable of binding with gold colloid particles was formed on the slide glass coated with the pattern control layer using trimethoxysilylpropyl(polyethyleneimine) (referred to as "PEIM", hereinafter) of formula 6 below. That is, 1 g of PEIM was dispersed in 99g of ethanol to prepare a coating solution. The coating solution was applied onto the slide glass manufactured in (1-a) by a microspotter (Biobobotics MicroGrid TAS) to form patterns on desired regions of the slide glass.

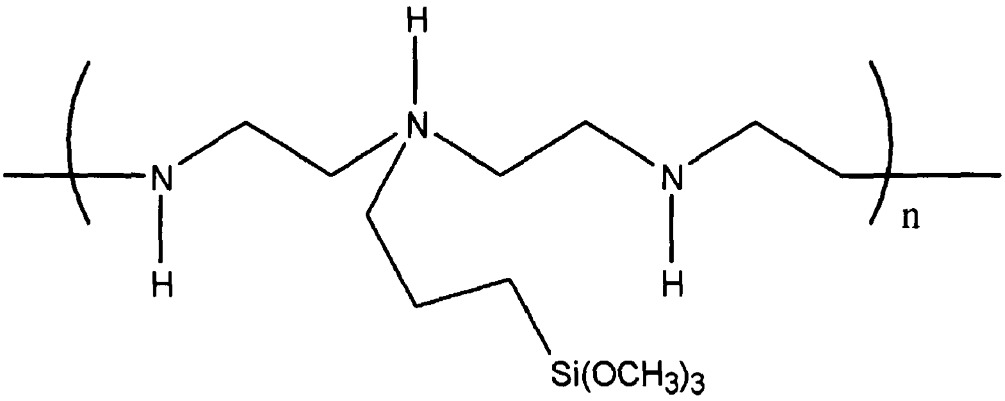

(6)

(1-c): Gold colloid reaction on pattern regions

A gold colloid solution was prepared as follows. 1 ml of 1 % $HAuCl_4 \cdot 3H_2O$ was added to 100 ml of demineralized water and heated with vigorously stirring. At 6 minutes after the heating was initiated, 2 ml of a 1% sodium citrate solution and 0.45 ml of a 1 % tannic acid solution were at a time added to the reaction solution. The resultant solution was stirred for one minute, cooled to room temperature, and stored at 4°C until use. At this time, gold particles had an average particle size of 5 nm. The resultant slide glass of (1-b) was dipped in the gold colloid solution for 30 minutes to coat the pattern regions with the gold colloid particles.

(1-d): Formation of gold pattern thin film by electroless plating

The resultant slide glass of (1-c) was dipped in a mixed solution of 1 ml of 1 % $HAuCl_4 \cdot 3H_2O$ and 10 ml of 0.4 mM $NH_2OH \cdot HCl$ for 10 minutes to form a desired gold thin film on the resultant slide glass by electroless plating.

Examples 2-5

Desired substrates for biomolecule immobilization were manufactured in the same manner as in Example 1 except that the weight ratio of the compound of formula 4 to the compound of formula 5 was 0.9:0.1 (Example 2), 0.5: 0.5 (Example 3), 0.3:0.7 (Example 4), and 0.1:0.9 (Example 5).

Examples 6-8

Desired substrates for biomolecule immobilization were manufactured in the same manner as in Example 1 except that the dipping time in the gold colloid solution was 5 minutes (Example 6), 10 minutes (Example 7), and 60 minutes (Example 8).

Example 9

A desired substrate for biomolecule immobilization was manufactured in the same manner as in Example 1 except that the gold particles in the gold colloid solution had an average particle size of 10 nm.

Example 10

The substrates for biomolecule immobilization manufactured in Examples 1-9 were immobilized with DNAs or proteins as biomolecules to manufacture biochips.

Comparative Example 1

A substrate for biomolecule immobilization was manufactured in the same manner as in Example 1 except that in (1-a) of Example 1, the compound of formula 5 was not used.

Comparative Example 2

A substrate for biomolecule immobilization was manufactured in the same manner as in Example 1 except that in (1-a) of Example 1, the compound of formula 4 was not used.

Experimental Example 1: Self-emission test for ion interaction layer patterns

Self-emission tests for the substrates for biomolecule immobilization manufactured in Examples 1-5 and Comparative Examples 1-2 were performed and the results are shown in FIG. 5. Scanning conditions using Laser 532 were as follows: Laser power 100 and PMT 500.

As shown in FIG. 5, when the weight ratio of the hydrophobic group-containing compound of formula 4 to the hydrophilic group-containing compound of formula 5 was 0.7:0.3 (Example 1), a self-emission level was the greatest. This shows that the PEIM compound of formula 6 used for an ion interaction layer pattern optimally binds with a pattern control layer at the above weight ratio of the compound of formula 4 to the compound of formula 5.

Experimental Example 2: Surface morphology evaluation after electroless plating

Surface states of the substrates after electroless plating according to Examples 1-5 and Comparative Examples 1-2 are shown in FIG. 6. As shown in FIG. 6, gold plating optimally occurred on the substrate of Example 1.

Experimental Example 3: Fluorescence test

The substrate of Example 1 after forming the PEIM pattern, after coating the gold colloid solution, and after electroless plating was scanned using Laser 532 under the following scanning condition: Laser power 100 and PMT 500, and the surface images of the substrate are shown in FIG. 7. As shown in FIG. 7, a self-emission level was reduced by forming the gold thin film on the substrate.

FIG. 8 is an optical microscopic image of the gold thin film pattern. Each pattern size was about 220 $\mu$m.

Experimental Example 4: Surface morphologies of gold thin films with respect to the reaction time between PEIM and gold colloid particles

Surface morphologies of the gold thin film patterns of Examples 1 and 6-8, with respect to the dipping time in the gold colloid solution are shown in FIG. 9. As shown in FIG. 9, as the reaction time increased, small particles were uniformly and densely arranged, thereby enhancing a substrate surface roughness.

Experimental Example 5: Particle growth with respect to the particle size of gold colloid particles

Images of the gold particles in the gold colloid solutions prepared in Examples 1 and 9 are shown in FIG. 10. As shown in FIG. 10, as the particle size of the gold colloid particles increased, a particle growth rate decreased.

According to the patterning method of the present invention, a fine pattern can be formed at room temperature under atmospheric pressure, and the shape and size of the pattern can be appropriately controlled to a desired level. Therefore, a desired substrate can be easily manufactured. When a biochip such as DNA chip or protein chip is manufactured by the above patterning method, immobilization of biomolecules on unwanted regions of a substrate can be prevented, and thus detection power can be improved.

## Claims

**1.** A patterning method comprising:

forming on a substrate a pattern control layer comprising a hydrophobic group-containing silane compound and a hydrophilic group-containing silane compound;
forming a selectively patterned ion interaction layer on the pattern control layer;
forming a seed colloid particle layer on the patterned ion interaction layer; and
growing a metal thin film from the seed colloid particle layer.

**2.** The patterning method of claim 1, wherein the hydrophobic group-containing silane compound is a compound represented by formula 1 below:

$$X\text{-}Si(R_1)_3, \qquad (1)$$

wherein X is a hydrophobic group, and $R_1$ is hydrogen, a substituted or unsubstituted alkoxy group of 1-20 carbon atoms, or halogen.

**3.** The patterning method of claim 2, wherein the hydrophobic group is a substituted or unsubstituted alkyl group of 1-20 carbon atoms or a substituted or unsubstituted aryl group of 6-30 carbon atoms.

**4.** The patterning method of claim 1, wherein the hydrophobic group-containing silane compound is octadecyltrichlorosilane, octadecyltriethoxysilane, phenyloxyundecyltrimethoxysi lane, or (heptadecafluoro-1,1,2,2-tetrahydrodecyl) triethoxysilane.

**5.** The patterning method of claim 1, wherein the hydrophilic group-containing silane compound is a compound represented by formula 2 below:

$$Y\text{-}Si(R_2)_3, \qquad (2)$$

wherein Y is a hydrophilic group, and $R_2$ is hydrogen, a substituted or unsubstituted alkoxy of 1-20 carbon atoms, or halogen.

**6.** The patterning method of claim 5, wherein the hydrophilic group is a substituted or unsubstituted hydroxyalkyl group of 1-20 carbon atoms, a substituted or unsubstituted carboxyalkyl group of 2-20 carbon atoms, a substituted or unsubstituted hydroxyalkylamino group of 1-20 carbon atoms, a substituted or unsubstituted hydroxyalkylaminoalkyl group of 2-20 carbon atoms, a substituted or unsubstituted di(hydroxyalkyl)aminoalkyl group of 3-20 carbon atoms, or a substituted or unsubstituted di(hydroxy)alkylaminoalkyl group of 2-20 carbon atoms.

**7.** The patterning method of claim 1, wherein the weight ratio of the hydrophobic group-containing silane compound to the hydrophilic group-containing silane compound ranges from 0.9:0.1 to 0.3:0.7.

**8.** The patterning method of claim 1, wherein the ion interaction layer comprises a compound represented by formula 3 below:

$$(R_3)_3\text{-}Si\text{-}Z, \qquad (3)$$

wherein $R_3$ is hydrogen, a substituted or unsubstituted alkoxy of 1-20 carbon atoms, or halogen, and Z is a positively charged functional group.

**9.** The patterning method of claim 1, wherein the ion interaction layer is formed on the pattern control layer by piezoelectric printing, micropipetting, inkjet printing, spotting, or stamping.

**10.** The patterning method of claim 1, wherein the seed colloid particle layer comprises gold colloid particles having an average particle size of 5 to 8 nm.

**11.** The patterning method of claim 1, wherein the metal thin film is a gold thin film.

**12.** The patterning method of claim 1, wherein the substrate is a glass, a silicon wafer, polycarbonate, polystyrene, or polyurethane.

**13.** The patterning method of claim 1, wherein forming the pattern control layer is performed using a coating composition including a pattern control layer forming compound composed of the hydrophobic group-containing silane compound and the hydrophilic group-containing silane compound and a coating solvent.

**14.** The patterning method of claim 1, wherein the coating composition is coated on the substrate by a wet coating method selected from the group consisting of dipping, spraying, spin-coating, and printing.

**15.** A substrate for biomolecule immobilization comprising:

a base substrate;
a pattern control layer, formed on the base substrate, controlling a surface tension;
a patterned ion interaction layer formed on the pattern control layer; and
a metal thin film selectively formed on the patterned ion interaction layer.

**16.** The substrate of claim 15, wherein the substrate has 1 to 10,000 biomolecule binding sites per $cm^2$ and each biomolecule binding site has a diameter of 50 to 5,000 $\mu$m.

**17.** A substrate for biomolecule immobilization comprising:

a base substrate having nanopores;
a pattern control layer, formed on the base substrate, controlling a surface tension;
an ion interaction layer, formed on the pattern control layer, being selectively patterned around the nanopores; and
a metal thin film selectively formed on the ion interaction layer patterned around the nanopores.

**18.** A biochip patterned by reacting and binding a biomolecule or a functional group-activated biomolecule with the metal thin film of the substrate of claim 15 or 17.

**19.** The biochip of claim 18, wherein the biomolecule is at least one selected from the group consisting of enzymes, proteins, antibodies, microorganism, animal cells and organs, plant cells and organs, nerve cells, DNAs, and RNAs, derived from living species or equivalents thereof, or synthesized ex *vivo*.

**20.** The biochip of claim 18, wherein the patterning of the biochip is performed by a patterning method selected from the group consisting of piezoelectric printing, micropipetting, stamping, and spotting.

FIG. 1

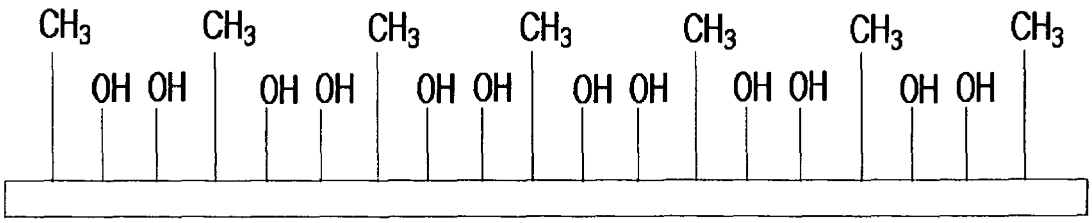

FIG. 2

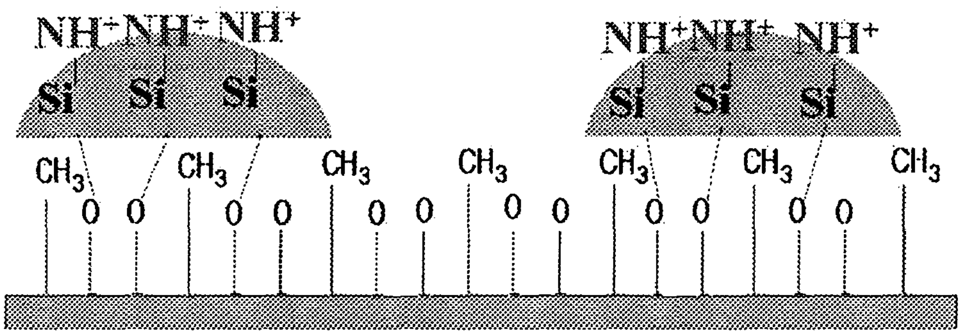

FIG. 3

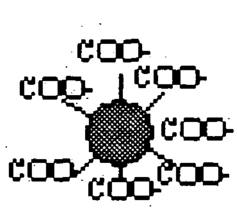

FIG. 4

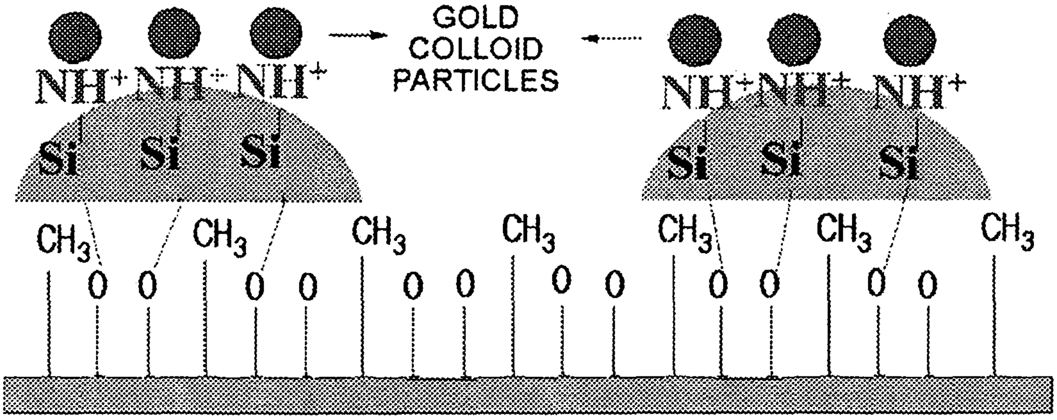
GOLD
COLLOID
PARTICLES
NH+ NH+ NH+
Si Si Si
CH3
O O

FIG. 5

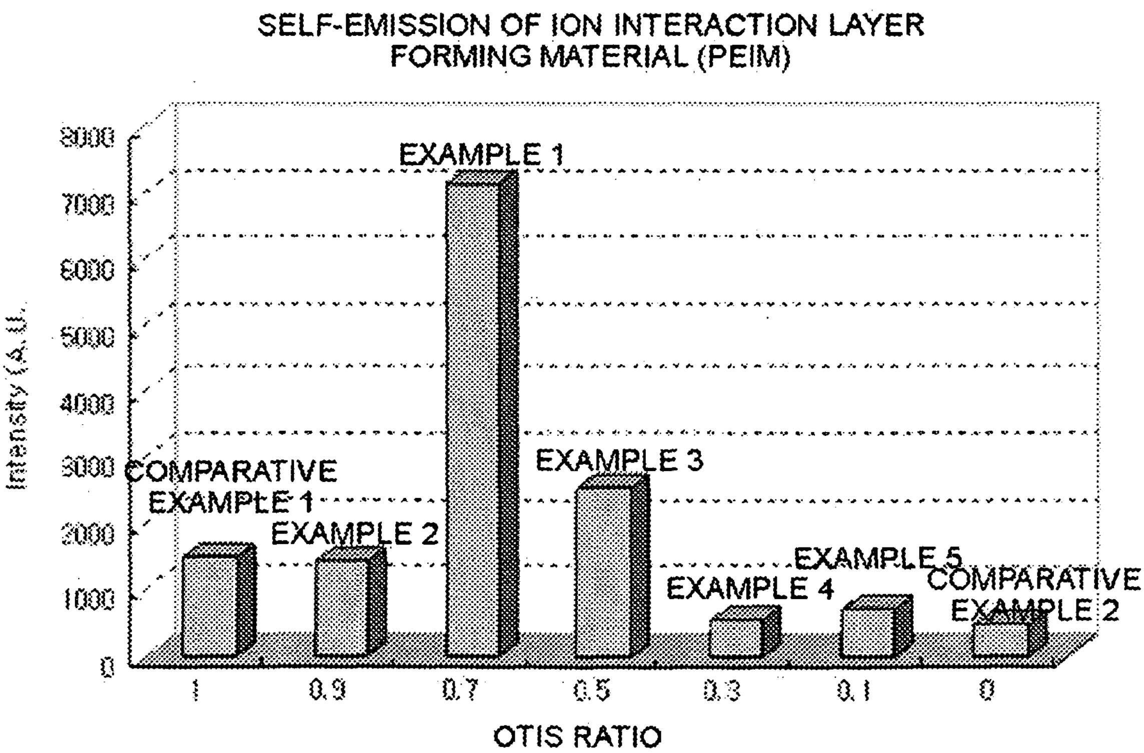
SELF-EMISSION OF ION INTERACTION LAYER
FORMING MATERIAL (PEIM)
Intensity (A.U.)
8000
7000
6000
5000
4000
3000
2000
1000
0
COMPARATIVE
EXAMPLE 1
EXAMPLE 2
EXAMPLE 1
EXAMPLE 3
EXAMPLE 4
EXAMPLE 5
COMPARATIVE
EXAMPLE 2
1
0.9
0.7
0.5
0.3
0.1
0
OTIS RATIO

FIG. 6

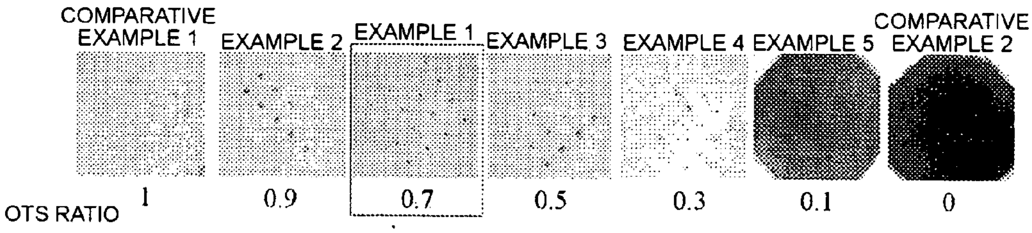


FIG. 7

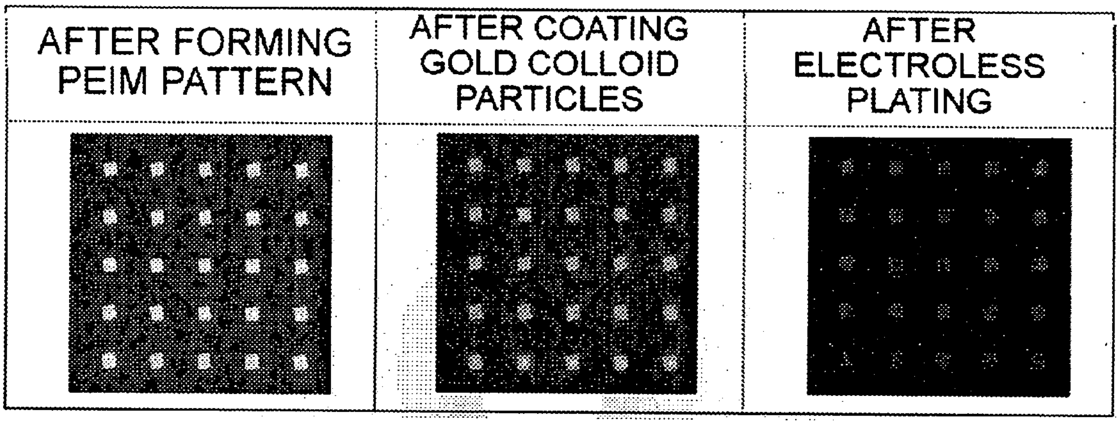


FIG. 8

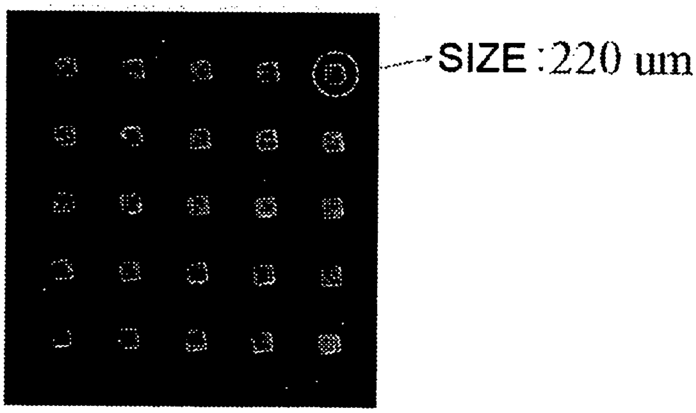

FIG. 9

EXAMPLE 6 EXAMPLE 7

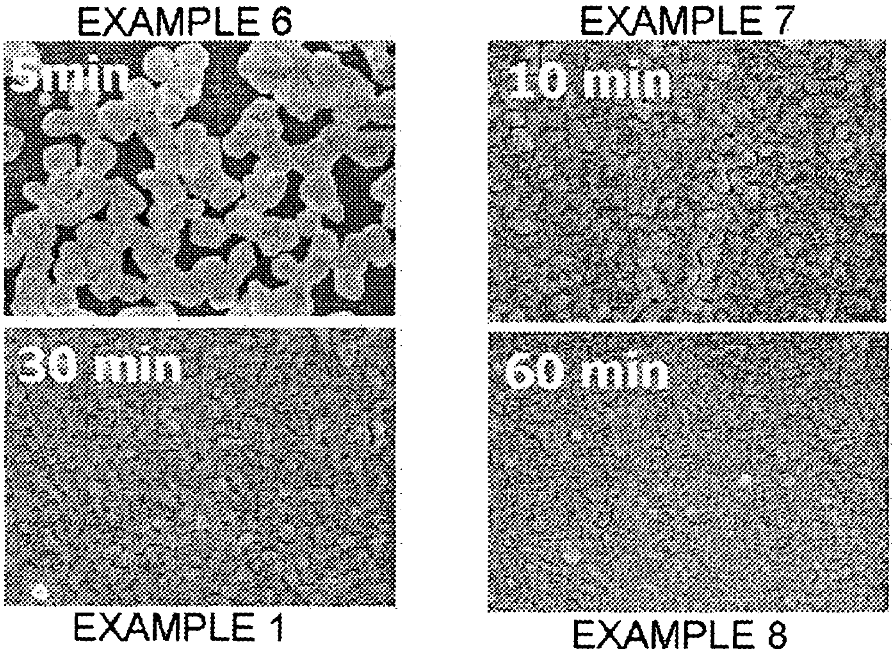


EXAMPLE 1 EXAMPLE 8

FIG. 10

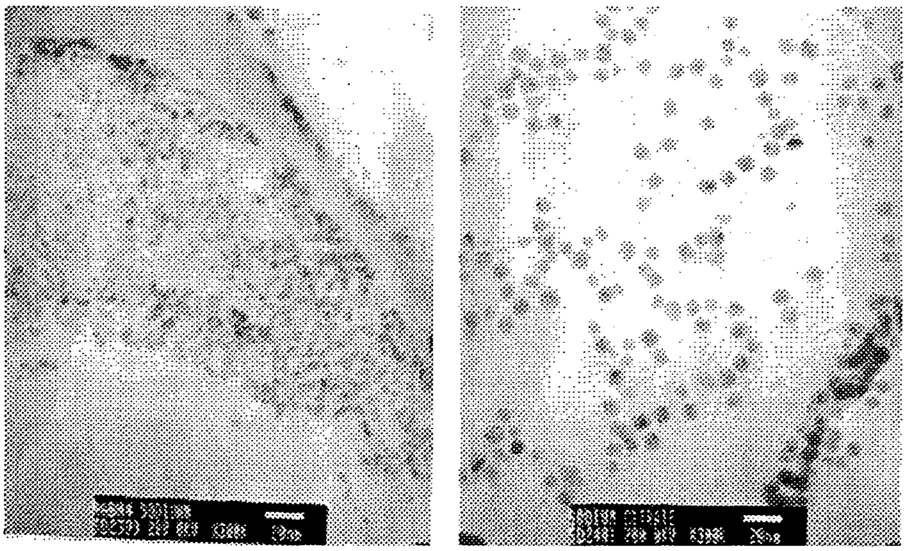

EXAMPLE 1: 5 nm EXAMPLE 9: 10 nm

European Patent Office

# EUROPEAN SEARCH REPORT

Application Number
EP 06 00 0607

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2001/012869 A1 (FUKUSHIMA MOTOO ET AL) 9 August 2001 (2001-08-09)<br>* abstract *<br>* paragraphs [0031] - [0033] *<br>* paragraphs [0039] - [0048]; example 1 *<br>----- | 1-15 | INV.<br>B01J19/00<br>G01N33/553<br>C23C18/16<br>H05K3/38 |
| X | US 6 355 198 B1 (KIM ENOCH ET AL) 12 March 2002 (2002-03-12)<br>* column 4, line 35 - line 38 *<br>* column 23, line 66 - column 24, line 12; figure 6b *<br>----- | 1-20 | |
| X | US 6 436 615 B1 (BRANDOW SUSAN L ET AL) 20 August 2002 (2002-08-20)<br>* examples 15,16,23,25 *<br>----- | 1-15 | |
| A | US 5 389 496 A (CALVERT ET AL) 14 February 1995 (1995-02-14)<br>* example 19 *<br>----- | 1-20 | |

TECHNICAL FIELDS SEARCHED (IPC)

B01J
G01N
C23C
H05K

The present search report has been drawn up for all claims

| Place of search | Date of completion of the search | Examiner |
|---|---|---|
| The Hague | 8 May 2006 | Veefkind, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4
EPO FORM 1503 03.82 (P04C01)

## ANNEX TO THE EUROPEAN SEARCH REPORT ON EUROPEAN PATENT APPLICATION NO.

EP 06 00 0607

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2006

| Patent document cited in search report | Publication date | Patent family member(s) | Publication date |
| --- | --- | --- | --- |
| US 2001012869 A1 | 09-08-2001 | NONE | |
| US 6355198 B1 | 12-03-2002 | NONE | |
| US 6436615 B1 | 20-08-2002 | AU 5636700 A<br>WO 0101199 A1 | 31-01-2001<br>04-01-2001 |
| US 5389496 A | 14-02-1995 | NONE | |

EPO FORM P0459

For more details about this annex : see Official Journal of the European Patent Office, No. 12/82